# EUROPEAN PATENT SPECIFICATION

(11) **EP 2 326 372 B1**
(45) Date of publication and mention of the grant of the patent: **18.09.2013**
(21) Application number: 09789081.8
(22) Date of filing: 05.08.2009
(51) Int. Cl.: A61M 5/32, F16F 1/04

(54) **SAFETY SYRINGE**
SICHERHEITSSPRITZE
SERINGUE DE SÉCURITÉ

(30) Priority: 14.08.2008 US 192014
(43) Date of publication of application: 01.06.2011
(73) Proprietor: Protectus Medical Devices, Inc., Minneapolis, MN 55413 (US)
(72) Inventor: DILLARD, John, A., B., II, Camarillo CA 93010 (US)
(74) Representative: Latham, Stuart Alexander
(86) International application number: PCT/US2009/004526
(87) International publication number: WO 2010/019201

(56) References cited:
- WO-A-2006/029003
- DE-U1-202007 001 717
- GB-A- 732 313
- US-A- 5 308 332
- US-A- 5 651 480

## Description

This invention relates to safety syringes and particularly to sliding sheaths moveable to cover needles on hypodermic syringes and has particular reference to the design of the spring and related components, the spring expanding to move the sheath forward, causing the sliding sheath to lock in a protective manner over the needle point or tip. The invention also relates to a unique configuration of a helical spring that has lesser turns and a greater strength when compressed than a comparable diameter prior art helical spring.

### BACKGROUND OF THE INVENTION

This invention is an improvement on the sliding sheath mechanism and the spring design for repositioning the sheath of the general type shown in U.S. Pat. No. 5,057,086 granted October 15, 1991, 5,279,584 granted January 18, 1994 and 5,308,332 granted May 3, 1994 to John A. B. Dillard III and James A. Orr.

The prior Dillard et al. patents disclose a sliding sheath designed to automatically cover the needle of a syringe if operator loses intentional control, or when the operator finishes injection/use. A ring latch, also referred to as a locking ring mechanism, maintains the sheath in its needle-covering position so that a person cannot accidentally prick himself or another person with the newly contaminated needle. The syringe sheath is propelled to its needle-covering position by a spring, preferably helical, carried on or attached to the exterior of the syringe body. During use of the syringe the operator manually grasps the locking ring or sheath and slides the sheath rearward, which results in compression of the spring to expose the needle point. When the operator has completed injection or use of the syringe, the locking ring or sheath is manually released and the spring propels the sliding sheath forward. If it is operating properly the end of the sheath slides past the point of the needle is then locked in the protecting position. As the sheath nears the forward end of the needle the latch mechanism interacts with the syringe body to latch the sheath in its needle-covering position. Normally, the operator completely releases the sheath, and the spring force moves the sheath forward and the sheath latch mechanism activates to lock the sheath in the needle-covering position

However, a problem can arise if the operator allows the spring to gently expand too extend the sheath over the needle. The last stages of spring expansion under this condition has such diminished force that it sometimes does not actuate the latch, and the sheath can then slide rearward under the impact of a blow, exposing the needle point. To alleviate this problem a stronger spring is necessary. However, to increase the strength of the spring in these prior designs the spring requires additional turns, must be thicker in cross-section or must be constructed from a different, stronger material.

A further problem of these prior devices is that in order to provide adequate expansive power, the spring has added turns, the added turns results in a longer collapsed length and, as a result, the collapsed spring length is too great to allow the full length exposure of the needle (i.e., the length from the needle point to the needle hub) to be utilized.

### BRIEF DESCRIPTION OF THE INVENTION

The spring can be made stronger by increasing its dimensions or using a different material of construction to get more terminal expansion force. However, this results in an increased size and more costly spring. It has now been discovered that the same material of construction can be employed if the spiral shape is modified according to claim 1. The spring has a significantly greater expansion force and a sufficient terminal force to positively actuate the latch mechanism even when the expansion is gently guided by the fingers of the operator. The locking mechanism has also been modified to provide a more secured locking structure.

### DESCRIPTION OF THE DRAWINGS

Referring to the drawings forming an integral part of this specification:

FIG. 1 is a cross-sectional side view of a prior art syringe as set forth in US Patent No. 5,308,332.

FIG. 2 is a cross-sectional view of a modified prior art form of the syringe of FIG. 1.

FIG. 3 is a cross-sectional view of the prior art syringe of FIG.2 with the sheath fully retracted.

FIG. 4 is a front view of a sheath spring incorporating features of the invention, other syringe components being shown in dotted lines.

FIG. 5 is a first front perspective view of the sheath spring of Fig 4 with the sheath shown in dotted lines.

FIG. 6 is a second perspective view of the sheath spring of Fig 4, taken at a rotation of 90° from the view in Figure 5, the sheath shown in dotted lines.

FIG. 7 is a cross-sectional view of a safety syringe incorporating features of the invention with the sheath retracted to fully expose the needle to the needle hub.

FIG. 8 is a cross-sectional view of a safety syringe of Fig 7 with the sheath in its extended and locked position covering the needle.

FIG. 9 is a side view of the locking ring which encloses the latching mechanism.

FIG. 10 is a longitudinal cross-sectional view of the locking ring of Fig. 9.

FIG. 11 is a cross-sectional view of the locking ring of Fig. 9 taken along line 11-11 of Fig. 10.

FIG. 12 is an enlarged view of the circled portion of Fig. 11.

FIG. 13 is a front perspective view of a one piece spring and sheath assembly incorporating features of the invention.

FIG. 14 is a cross-sectional view looking rearward of the sheath and latch base taken along line 14-14 of Fig. 13.

FIGS. 15A, 15B and 15C are cross-sectional views looking rearward of the locking ring and sheath taken along line 15-15 of Fig 8, with the locking ring rotated relative to the sheath to lock the sheath in its forward position.

FIG. 16 is an enlarged view of the circled portion 316 of Fig. 8.

FIG. 17 is an enlarged view of the circled portion 317 of Fig. 7, said view encompassing the same location on the syringe as in Fig. 16, showing both the plunger tip in its forward most position and the sheath in its forward most locked position.

FIG. 18 is a top view of the spring of Figs. 5 and 6.

FIGS. 19, 20, 21 and 22 are orthogonal views of the spring portion of Figs. 5 and 6; each successive view rotated 90° from the prior view to provide front, right side, rear and left side views.

FIG. 23 is a bottom view of the spring of Figs. 5 and 6.

FIG. 24 shows a second embodiment of the spring and sheath provided as separate attachable components.

FIG. 25 is a top view of the spring of Fig. 24.

FIGS. 26, 27, 28 and 29 are orthogonal views of the spring portion of Fig. 24; each successive view rotated 90° from the prior view to provide front, right side, rear and left side views.

FIG.30 is a bottom view of the spring of Fig. 24.

FIG. 31 is a longitudinal cross-sectional view of the embodiment of Fig 24.

### DETAILED DESCRIPTION OF THE DRAWINGS

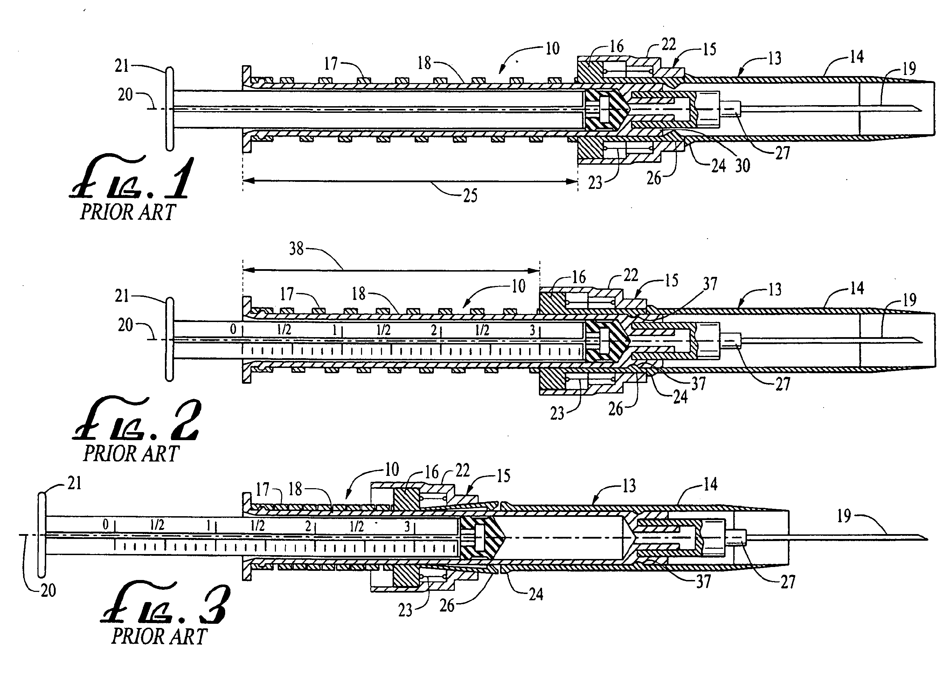

Reference is made to the prior art of FIG. 1-3 which is a safety hypodermic syringe 10 shown in US Pat. No. 5,308,332. In the description of the hypodermic syringe 10, the needle end is referred to as the forward end, movement of a component toward the needle end is referred to as "forward" and movement of a component in a direction away from the forward end is referred to as "rearward". The syringe 10 includes a sheath assembly 13 which includes a reciprocal sheath 14, a latch base 16, and a sheath spring 17. These three parts were of a unitary construction formed of a single piece of material, such as an injection molded plastic. The sheath 14 reciprocates over a hollow syringe body 18 and a hollow needle 19 which is connected to the forward end of the syringe body 18. A manually operated plunger 21 is located within the syringe body. When there is liquid in the syringe body 18, manually pressing the plunger 21 results in the liquid in the syringe body 18 being driven through the hollow needle 19.

The syringe 10 has a longitudinal axis 20 through the syringe body 18 and needle 19. A latch mechanism 15 comprises a reciprocating locking ring 22, which is urged to a forward position by a latch spring 23 which is between the locking ring 22 and over the rear end of the sheath 14, one end of the latch spring 23 bearing against the latch base 16 with the other end bearing against an inner end surface of the locking ring 22. The movement of the locking ring 22 to the forward position is halted by tabs 24 which are integral with but extend outward from the sheath 14.

The latching action on the prior art devices is accomplished by a pair of latch fingers 26 that normally spring radially outward, but are forced radially inward by the forward end of the locking ring 22 when it is moved to the forward position as shown in FIG. 1. These fingers 26 are integrally connected to the sheath 14. When they are forced inwardly as shown in FIG. 1, they extend past and contact the forward end 30 of the hollow syringe body 18, to prevent the sheath 14 from moving rearward. The latch spring 23 normally urges the locking ring 22 forward, which holds the sheath 14 in its extended position as shown in FIG. 1.

After the contents of the syringe 10 are injected and the needle 19 is withdrawn from the puncture site the sheath spring 17 is supposed to drive the sheath 14 forward so that it covers the needle 19 as shown in FIG 1. The latch spring 23 then moves the locking ring 22 forward, forcing the fingers 26 inward to hold the sheath 14 in its extended or covering position. This construction generally prevents the sheath from being retracted unless considerable force is intentionally applied to the structure to defeat the locking safety features. Therefore, if the syringe 10 contacts other persons, they are protected from injury by the shielded needle 19.

Referring to FIG. 1, the sheath spring 17 is shown in its fully extended condition, the length of the fully extended condition being referred to as the free expansion dimension 25. To use the syringe the sheath 14 is retracted. After delivery of the contents of the syringe 10, the operator releases the locking ring 22 and the sheath spring 17 moves the sheath 14 forward causing the latch base 16 and locking ring 22 to move forward so that the sheath 14 covers the needle 19 as shown. The latch fingers 26 are located forward of the forward end 30 of the syringe body 18. The locking ring 22 is forced to its forward position by the latch spring 23, moving the latch fingers 26 inwardly to the configuration as shown in Fig 1.

However, in some instances, whether the operator through inadvertence or otherwise allows the sheath spring 17 to only slowly expand or something interferes with the expansion of the sheath spring 17 to its fully expanded dimension 25, experience shows that friction during the last 5% or 10% of the movement will reduce expansion energy of the sheath spring 17 to an extent that the locking function will not properly operate and the latch fingers 26 will not be positioned beyond the forward end 30 of the syringe barrel. In this event the sheath 14 will not move into its locked position and the needle point can be inadvertently exposed, potential injuring the personnel present.

One prior modification was to place a groove 37 at a position rearward of end of the syringe body 18. so that the sheath spring 17 would not have to extend to the same extent to latch. Referring to the prior art device of FIGS. 2 and 3, an annular groove 37 is formed near the forward end of the syringe body 18 to receive the latch fingers 26. The forward movement of the locking ring 22 is limited by raised tab 24, formed from material extending outwardly from the surface of the sheath 14. As a result, the sheath 14 of Fig. 2 must be made longer than the sheath 14 of FIG. 1 to accommodate this change in latching position of the latch fingers 26 and still adequately cover the needle point.

One result of this change is to restrict the expansion of sheath spring 17 to a length referred to as the "Restricted Expansion Dimension" 38. While it was found that this construction improved the locking operation it appears that the spring expansion strength was still not adequate and, as shown in FIG. 3, the number of turns in the spiral spring still prevented adequate retraction of the sheath and full exposure of the shaft of the needle 19. FIG. 3 shows the device of FIG. 2 in its fully retracted position. Because of the added length of the sheath 14 the full length of the needle 19 is not usable, i.e., the forward end of the needle hub 27 is not exposed.

To avoid the possibility that the sheath does not enter the locked position, another alternative is that the sheath spring 17 can be made stronger by increasing its cross-sectional dimensions or by adding one or more turns to the spiral. However, this solution adds weight and cost to the syringe 10 and increases the compressed length of the spring such that the sheath 14 does not fully retract and restricts insertion of the needle 19 through the puncture site to its full length (i.e., up to the needle hub 27). Applicant has discovered that the necessary force to overcome this non-latching can be obtained by replacing the sheath spring 17 with the unique spring structure shown and described herein below.

It has now been found that prior designs had a problem providing both the ability to withdraw the sheath sufficiently to expose the full length of the needle 19 from its point to the hub 27 and, when the sheath 14 is released, to insure that the needle point is sufficiently covered and the sheath is locked in its forward, needle covering position. This problem has been eliminated by changing the configuration of the spring. The prior art devices such as shown in Figs. 1-3 included a molded, helical, polycarbonate plastic expansion spring 17 with a uniform spiral configuration. In other words, the coil of the spring forms a three-dimensional curve along a cylindrical surface, such that its angle to a plane perpendicular to the longitudinal axis 20 of the cylinder (i.e., the syringe body 18) is constant. The molded spring comprises a rectangular cross-section (approximately 0.25cm (0.1 in.) by 0.89cm (0.35 in)) plastic coil with approximately eight turns, each turn being uniformly spaced from the adjacent turn. In its expanded configuration it is approximately 6.3 cm long (the free expanded dimension 25) and when fully compressed it has a length of approximately 2.8 cm. This allows the sheath to be retracted approximately 3.5 cm. When fully compressed it expands with a force of about 4N (0.9 pounds).

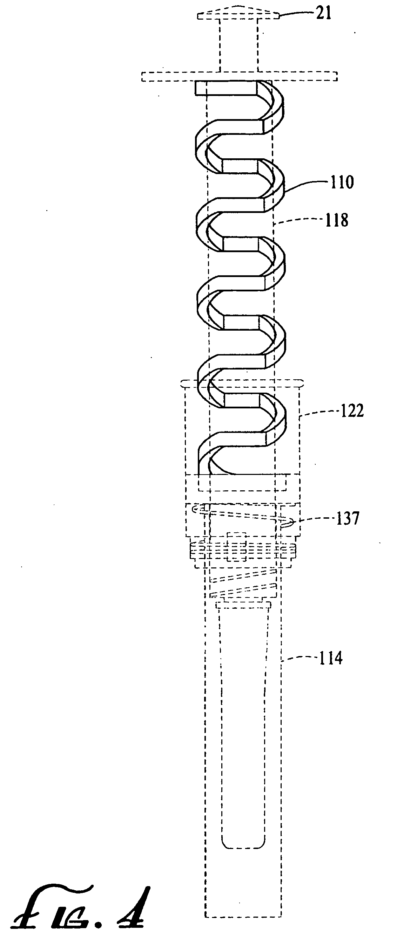

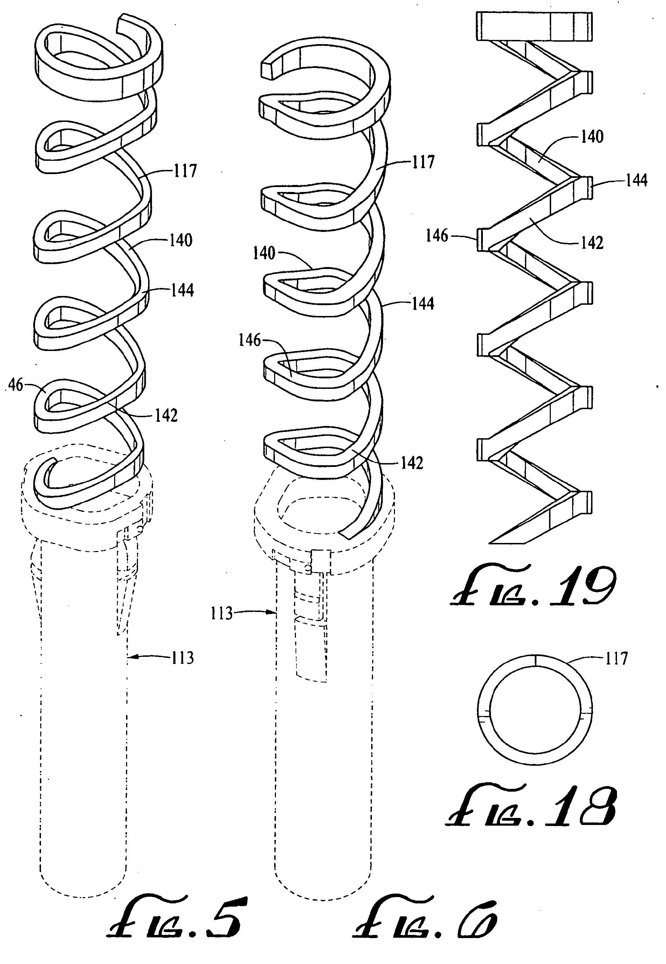

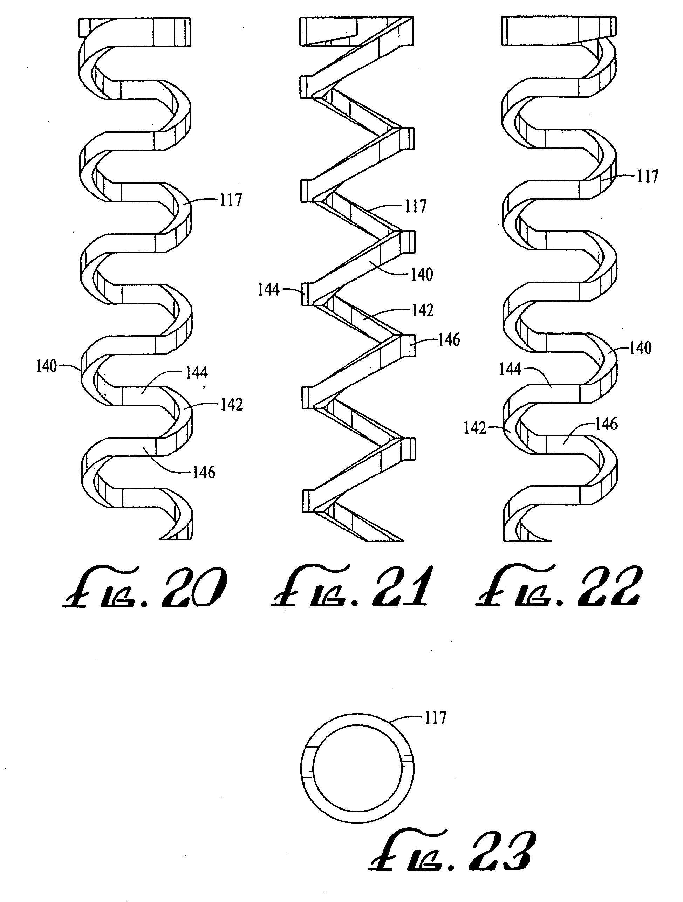

In contrast, a non-uniform spring 117 incorporating features of the invention utilizes a molded, helical, polycarbonate plastic with substantially the same cross-section as the prior art spring 17 and the same polycarbonate material. However, while the turns of the spiral are uniformly spaced from the adjacent turns, the spiral is non-uniform. In other words, the coil of the spring forms a three-dimensional curve along a cylindrical surface, such that its angle to a plane perpendicular to the longitudinal axis 120 of the syringe body 1.8 is not constant. Referring to Figures 4 - 6, 13, 19 - 22, 24-29 and 31 and particularly FIG. 19 in a single continuous 360° turn of the spiral, the turn comprises two portions which are at the same angle to a plane perpendicular to the axis of the cylinder (referred to as first and second angled portions 140, 142) and two portions approximately parallel to a plane perpendicular to the axis of the cylinder (referred to as first and second flat portions 144, 146) the angled and flat portions alternating along the length of the spiral. As an example of a suitable construction the non-uniform spiral has a first angled portion 140 for about 120-140° of rotate, a first flat portion 144 for about 40-60° of rotate, a second angled portion 142 for about 120-140° of rotate and a second flat portion 146 for about 40-60° of rotate. This is then repeated for subsequent turns along the length of the non-uniform spiral. The angled portion 140, 142 more preferably constitute 125-135° of rotation, most preferably about 130° of rotation with the flat portions 144, 146 constituting 45-55° of rotation, most preferably about 50° of rotation. However, based on the teachings herein one skilled in the art can adjust the spring tension by adding or reducing the number of turns, changing the angle of rotation occupied by the angle and flat portions, adding additional flat and angled portions within a single turn having only one flat and one angled portion within a single turn, or providing the flat portion at other than approximately parallel to a plane perpendicular to the axis of the syringe body 118, for example, at an angle greater then or less than parallel as long as each successive 360° turn has the same shape to allow complete collapse of each turn against successive turns.

In the embodiment shown the non-uniform spring 117 has approximately 5.5 turns, in its expanded configuration it is approximately 6.6 cm long (the free expanded dimension 25) and when fully compressed it has a length of approximately 2.1 cm. This allows the sheath to be retracted approximately 4.5 cm. When fully compressed it expands to more than three times its compressed length with a force of about 5.8N (1.3 pounds). The significantly increased expansion force (approximately 45% greater) is a result of the non-uniform spiral shape and the significantly increased expanded length when compared to compressed length (approximately 29%) is a result of the fewer turns in the spring. As a result the sheathed syringe 110 is lighter in weight, requires less polymer to form the syringe, the same length sheath 14 can be withdrawn further to expose a longer needle length allowing better placement into the puncture site, and the increased spring tension allows a more positive locking of the sheath in its protective position after use.

Further, while the design of each 360° turn is described as having angled portions 140,142 and flat portions 144,146, the invention also contemplates alternative portions with different angles to the plane parallel to the longitudinal axis 120. In the embodiment disclosed, the angled portions 140,142 are at an angle of from about 22° to about 45° to the plane, preferably about 33° to the plane and the flat portions 144,146 are parallel to the plane (i.e., at an angle of about 90° to the axis 120). An alternative with two sets of angled portions can, for example, have a first set at the same angle (i.e., 22° to 45°) and a second set at a lesser angle (i.e., 0° to 20°) which may also be at a negative angle (i.e., 0° to 20°). However, the specific disclosed angles are exemplary and not limiting, the distinction being that there is a difference between the angles in the two alternating portions. Irrespective of the combination of angled portions in each 360° turn, each adjacent and successive turn of the helical spring repeats the combination of angled portions.

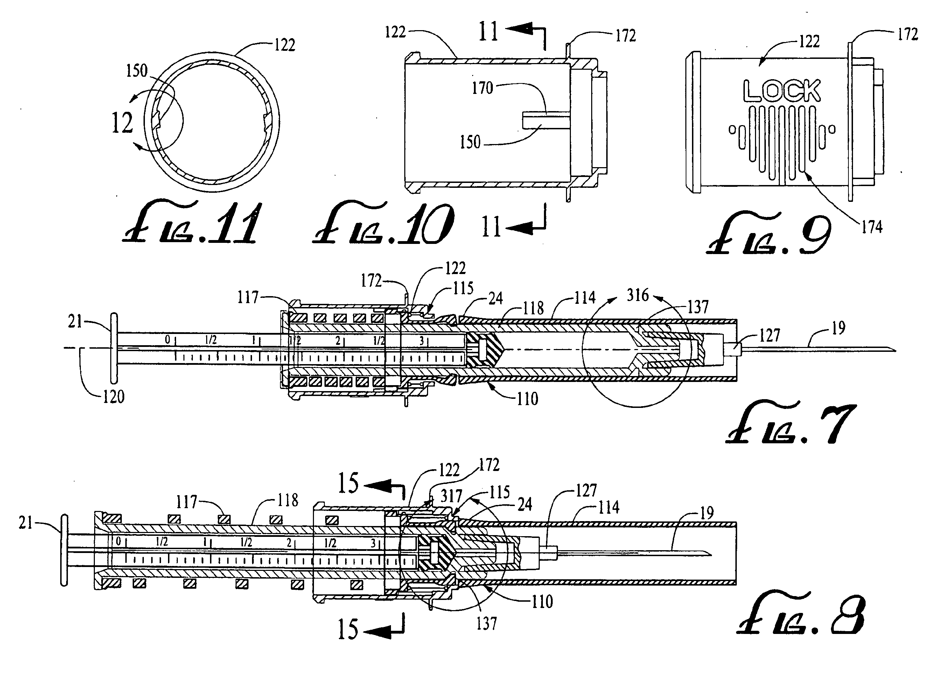

Figures 7 and 8 are a cross sectional views of a sheathed syringe 110 incorporating the above described non-uniform helical spring 117, as evidenced by the spring 117 having far fewer turns then the spring 17 in the prior art devices of Figures 1-3. Figure 7 shows the sheath 114 in its fully retracted position with the needle hub 127 extending beyond the forward end of the retracted sheath 114. Figure 8 shows the sheath 114 in its fully extended, locked position.

Also shown in Figures 7 and 8 are cross sections of the latch mechanism 115. Figures 9 and 10 are an enlarged side view and a longitudinal cross-section view respectively of the locking ring 122 of the latching mechanism and Figure 11 is a view taken along line 11-11 of Figure 10. Within circled portion 12 of Figure 11 is one of the two locking tabs 150 on the inner surface of the locking ring 122.

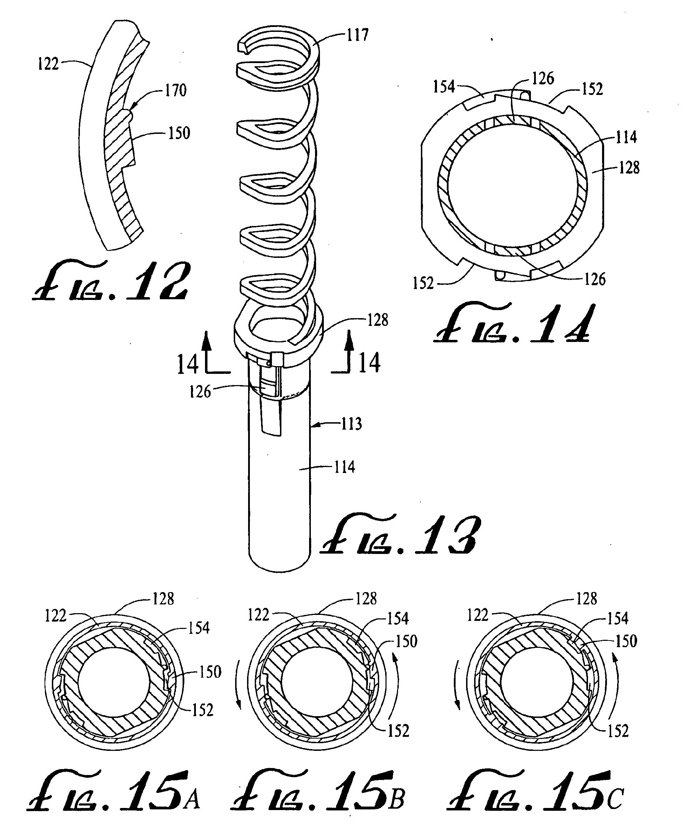

Figure 12 is an enlarged view of the circled portion 12 better illustrating one design for the locking tab 150. As described below, the locking tabs 150 interact with grooves, notches or extensions 152 on or in the outer surface of the sheath base 128 so that when the sheath 114 is in its forward position and the locking ring 122 is rotated to its locking position, the latch fingers 126, which are then resting in the groove 137, are locked into that position to prevent inadvertent rearward movement of the sheath 114. To illustrate this locking procedure, reference is made to Figure 14, which is a cross-section of Figure 13 at line 14-14 looking rearward and Figures 15A, 15B, and 15C taken along line 15-15 of Figure 8 which are cross-sectional views showing the locking ring 122 in three different rotational orientations. In Figure 15A, the syringe is shown prior to delivery of its contents with the locking tab 150 resting in a groove 152 on the sheath base 128. To withdraw the sheath 114 for delivery of its contents the user grasps the locking ring 122, the radial extension 172 thereon or sheath 114 and moves it rearward compressing the non-uniform spring 117 and depresses the plunger 21. After delivery of the syringe contents the needle 19 is withdrawn from the injection site, the locking ring 122 or sheath 114 is released and the sheath 114, driven by the spring 117, moves forward to cover the point of the needle 19. The locking ring is then manually rotated, preferably clockwise, so the locking tab 150 moves out of the pass through groove 152 as shown in Figure 15B over the rib 170 and then into the locking channel 154 as shown in Figure 15C to place the sheath in a safe (locked) position. The direction of rotation to effect locking can be shown by an arrow 174 molded into the surface of the locking ring. In the safe position the front end of the locking ring 122 is biased forward by the latch spring 23 so that it rests directly over the latch fingers 126 to hold them in the groove 137 to retard or prevent unintended rearward movement of the sheath. To aid in visualizing that the locking ring is moved forward into its locking position over the latch fingers 126, the latching fingers can be colored, for example be provided with a red appearance. If the locking ring is not fully forward the color of the latching fingers 126 is visible forward of the front edge of the locking ring 122. However, when the locking ring is advanced to its forward most position by the latch spring 23 the colored latching fingers are no longer visible, indicating that the sheath is now in its safe mode.

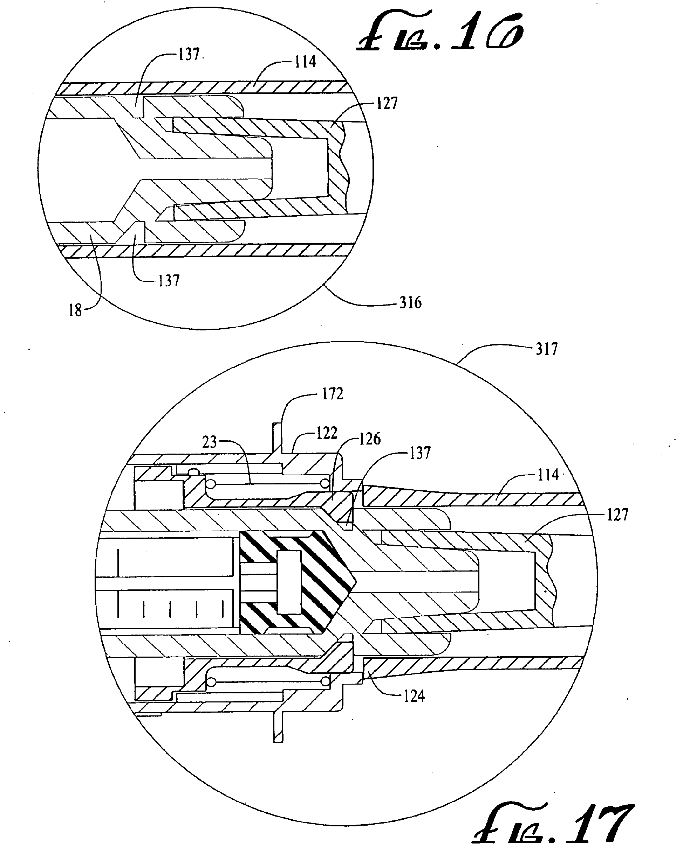

Figure 16 is an enlarged view of a portion of Figure 7 enclosed within the circled area 316 to better illustrate the groove 137 on the forward end of the syringe 110 to receive the latch fingers 126. Figure 17 is an enlarged view of a portion of Figure 7 enclosed within the circled area 317 to better illustrate the end of the latch fingers 126 resting within the groove 137 on the forward end of the syringe 110 and held within the groove 137 by the front edge of the locking ring 122, now resting against the raised tab 124 on the sheath 114 surface.

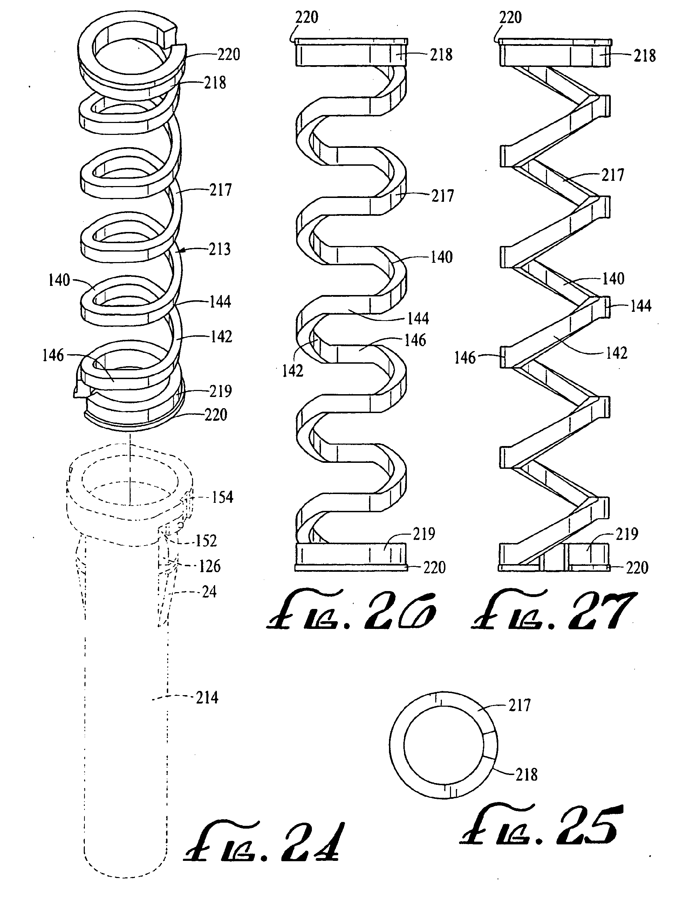

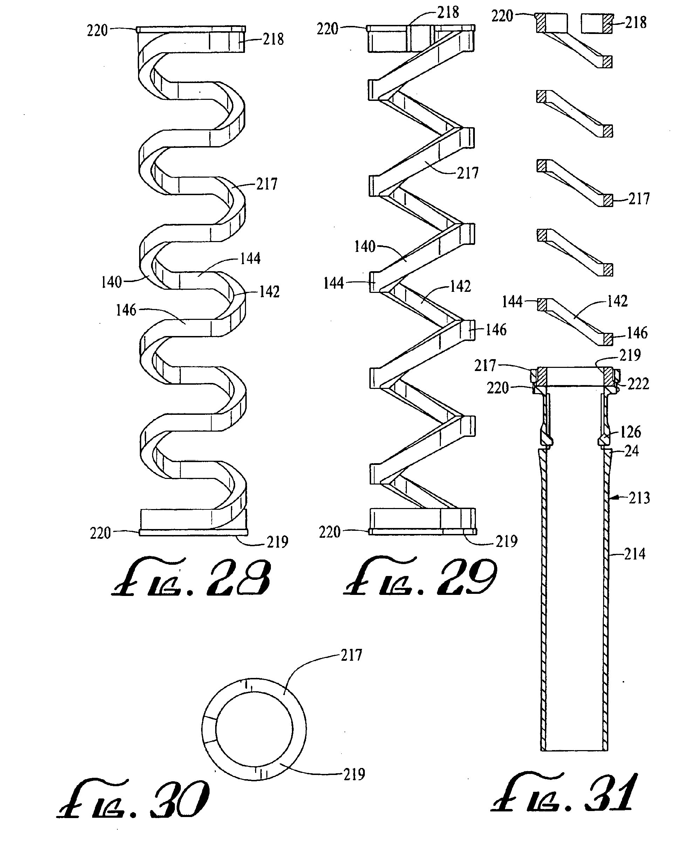

Figure 13 illustrates a first embodiment of the sheath assembly 113 wherein the non-uniform helical spring 117 is integral with the sheath 114. Figures 4, 5 and 6 shows the same embodiment with the sheath and other portions of the safety syringes showed in the dotted lines. Figures 18-23 show several different views of the non-uniform spring portion 217 of the sheath assembly 213. In an alternative embodiment of the sheath assembly 213 the non-uniform helical spring 217 can be fabricated separate from the sheath 214 and the two components joined by known plastic joining techniques. Figures 24-31 show several different views of the non-uniform helical spring 217 as a separate component from the sheath 214. Figure 31 is a cutaway side view showing the non-uniform helical spring 217 attached to the sheath 214. A preferred method of joining the spring 217 with the sheath 214 is to form the spring with flat top and bottom ends 218, 219, each end having an enlarged circumferential rim 220 on each of the flat ends 218, 219. The inner surface of the top end of the sheath 214 has a rim-receiving groove 222 so that the pieces can be snapped together. Examples of other joining techniques include, but are not limited to, adhesive or solvent bonding, heat bonding, tack welding, compression assembly and laser bonding.

The safety syringe and the unique non-uniform helical spring have been described with respect to the presently preferred embodiments. Various modifications and improvements will be apparent to those skilled in the art. All such variations, modifications, changes, and improvements that come within the scope of the invention are included within the scope of the attached claims.

## Claims

1. A helical compression/expansion spring comprising a non-uniform helical spring (117,217) having multiple 360° turns each turn uniformly spaced from adjacent turns, each 360° turn comprising alternating first angled portions (140) and second angled portions (142), the first angled portions (140) being at a first angle to a plane perpendicular to an axis (20) through the center of the helical spring (117,217) and the second angled portions (142) being at a second angle to the plane perpendicular to the axis (20), the first angle and the second angle being different.

2. A helical compression/expansion spring (117,217) according to claim 1, wherein the second angled portions (142) are flat portions (144,146), the flat portions being substantially parallel to the plane perpendicular to the axis.

3. The helical compression/expansion spring (117,217) of claim 2 wherein each 360° turn of the helical spring has a first and second angled portion (140,142) alternating with a first and second flat portion (144,146).

4. The helical compression/expansion spring (117,217) of claim 3 wherein the helical spring comprises five to six 360° turns.

5. The helical compression/expansion spring (117,217) of claim 4 wherein the helical spring when fully compressed has a length of about 2.1cm and an expanded length of about 6.6cm.

6. The helical compression/expansion spring (117,217) of claim 4 wherein the spring when fully compressed has an expansion force of about 5.8N (1.3 pounds).

7. The helical compression/expansion spring (117,217) of claim 4 wherein the fully compressed spring when released expands to more than 3 times its compressed length.

8. An improved safety hypodermic syringe (10) having:
a) a hollow tubular syringe body (18),
b) a hollow needle (19) communicating with the hollow of the syringe body (10),
c) a reciprocal tubular needle sheath (14) disposed on the exterior of the syringe body (18),
d) a spring (117,217) engaging the syringe body (10) and the sheath (14) and expandable to move the sheath (14) to cover the needle point (19), and
e) a latch mechanism (15) engaging the syringe body (10) and the sheath (14) to latch the sheath (14) in a needle-covering position after delivering contents from the syringe body (10),
wherein the improvement comprises a non-uniform helical spring (117,217) according to any of claims 2 to 7.

9. The improved safety syringe (10) of claim 8 wherein the spring (117,217) and the sheath (14) comprise a unitarycomponent.

10. The improved safety syringe (10) of claim 9 wherein the spring (117,217) and sheath (14) are separate components joined to form a unitary component.

11. The improved safety hypodermic syringe (16) of claim 8 wherein the latch mechanism (15) comprises a rotatable locking ring (22) with an internal extension (152) configured to engage with a groove (37, 137, 152) on the base of the sheath (14) so as to secure latching fingers (126) integral with the sheath (14) into a groove (37, 137, 152) on the syringe body (18) adjacent a hub (27) end of the hollow needle (19).

12. The improved safety hypodermic syringe(117,217) of claim 8 wherein the latch mechanism (15) includes a locking ring (22) in surrounding relationship to the sheath and the syringe, the locking ring having a longitudinal rib (170) on an inside surface thereof, said rib (170) interacting with a pass-through groove (152) and locking channel (154) on the base of the sheath (14) such that the locking ring (22) is positioned over latching fingers (126) integral with the sheath (19) surface to hold the latching fingers (126) in a groove (152) in the syringe surface rearward of the needle hub (27) to prevent movement of the sheath (24) once locked in the needle-covering position.

13. The improved safety hypodermic syringe (117,217) of claim 12 wherein locking ring (22) includes a radially extended edge on a forward outer surface thereof for grasping the locking ring (22) for retraction of the sheath (14).

14. The improved safety hypodermic syringe (117,217) of claim 12 wherein the latching fingers (126) are coloured to aid in visualizing that the sheath (14) is locked in the needle-covering position.

15. The improved safety hypodermic syringe (117,217) of claim 12 wherein the locking ring (22) includes an arrow (174) on the surface thereof to indicate the radial direction to turn the locking ring (22) to permanently lock the sheath (14) in its needle-covering position.

## Patentansprüche

1. Kompressions-/Expansionsschraubenfeder, umfassend eine ungleichmäßige Schraubenfeder (117, 217), die mehrere 360°-Windungen aufweist, wobei jede Windung von benachbarten Windungen gleichmäßig beabstandet ist, wobei jede 360°-Windung abwechselnd erste winkelige Teilbereiche (140) und zweite winkelige Teilbereiche (142) umfasst, wobei die ersten winkeligen Teilbereiche (140) sich in einem ersten Winkel zu einer Ebene senkrecht zu einer Achse (20) durch das Zentrum der Schraubenfeder (117, 217) befinden und die zweiten winkeligen Teilbereiche (142) sich in einem zweiten Winkel zur Ebene senkrecht zur Achse (20) befindet, wobei der erste Winkel und der zweite Winkel unterschiedlich sind.

2. Kompressions-/Expansionsschraubenfeder (117, 217) nach Anspruch 1, wobei die zweiten winkeligen Teilbereiche (142) flache Teilbereiche (144, 146) sind, wobei die flachen Teilbereiche im Wesentlichen parallel zur Ebene senkrecht zur Achse sind.

3. Kompressions-/Expansionsschraubenfeder (117, 217) nach Anspruch 2, wobei jede 360°-Windung der Schraubenfeder einen ersten und zweiten winkeligen Teilbereich (140, 142) aufweist, der sich mit einem ersten und zweiten flachen Teilbereich (144, 146) abwechselt.

4. Kompressions-/Expansionsschraubenfeder (117, 217) nach Anspruch 3, wobei die Schraubenfeder fünf bis sechs 360°-Windungen umfasst.

5. Kompressions-/Expansionsschraubenfeder (117, 217) nach Anspruch 4, wobei die Schraubenfeder, wenn vollständig komprimiert, eine Länge von etwa 2,1 cm und eine expandierte Länge von etwa 6,6 cm aufweist.

6. Kompressions-/Expansionsschraubenfeder (117, 217) nach Anspruch 4, wobei die Feder, wenn vollständig komprimiert, eine Expansionskraft von etwa 5,8 N (1,3 Pounds) aufweist.

7. Kompressions-/Expansionsschraubenfeder (117, 217) nach Anspruch 4, wobei sich die vollständig komprimierte Feder auf mehr als das 3-fache seiner komprimierten Länge ausdehnt, wenn sie entspannt wird.

8. Verbesserte Sicherheitssubkutanspritze (10), aufweisend:
a) einen hohlen, röhrenförmigen Spritzenkörper (18),
b) eine Hohlnadel (19), die mit dem Hohlraum des Spritzenkörpers (10) in Verbindung steht,
c) eine wechselseitige, röhrenförmige Nadelschutzhülle (14), die außen am Spritzenkörper (18) angebracht ist,
d) eine Feder (117, 217), die mit dem Spritzenkörper (10) und der Schutzhülle (14) in Eingriff steht und expandierbar ist, um die Schutzhülle (14) zu bewegen, so dass sie die Nadelspitze (19) abdeckt, und
e) einen Verriegelungsmechanismus (15), der mit dem Spritzenkörper (10) und der Schutzhülle (14) in einer die Nadel abdeckenden Position in Eingriff steht, nachdem Inhalte aus dem Spritzenkörper (10) abgegeben wurden,
wobei die Verbesserung eine ungleichmäßige Schraubenfeder (117, 217) gemäß einem der Ansprüche 2 bis 7 umfasst.

9. Verbesserte Sicherheitsspritze (10) nach Anspruch 8, wobei die Feder (117, 217) und die Schutzhülle (14) eine Einheitskomponente umfassen.

10. Verbesserte Sicherheitsspritze (10) nach Anspruch 9, wobei die Feder (117, 217) und die Schutzhülle (14) separate Komponenten sind, die miteinander verbunden sind, so dass sie eine Einheitskomponente bilden.

11. Verbesserte Sicherheitssubkutanspritze (16) nach Anspruch 8, wobei der Verriegelungsmechanismus (15) einen drehbaren Feststellring (22) mit einer inneren Ausweitung (152), die dazu geeignet ist, mit einer Auskehlung (37, 137, 152) an der Basis der Schutzhülle (14) in Eingriff zu kommen, so dass mit der Schutzhülle (14) integrale Verriegelungsfinger (126) in einer Auskehlung (37, 137, 152) am Spritzenkörper (18) neben einem Lagerende (27) der Hohlnadel (19) befestigt werden.

12. Verbesserte Sicherheitssubkutanspritze (117, 217) nach Anspruch 8, wobei der Verriegelungsmechanismus (15) einen Feststellring (22) in umgebender Anordnungsbeziehung zur Schutzhülle und Spritze enthält, wobei der Feststellring eine Längsrippe (170) an einer Innenfläche davon aufweist, wobei die Rippe (170) mit einer Durchgangsauskehlung (152) und einem Verriegelungskanal (154) an der Basis der Schutzhülle (14) derart interagiert, dass der Feststellring (22) über mit der Schutzhüllenoberfläche (19) integralen Verriegelungsfingern (126) positioniert wird, so dass die Verriegelungsfinger (126) in einer Auskehlung (152) in der Spritzenoberfläche hinter dem Nadellager (27) gehalten werden, um eine Bewegung der Schutzhülle (24) zu verhindern, sobald er in der die Nadel abdeckenden Position arretiert ist.

13. Verbesserte Sicherheitssubkutanspritze (117, 217) nach Anspruch 12, wobei der Feststellring (22) eine radial erweiterte Kante an einer vorderen Außenfläche davon enthält, um den Feststellring (22) zum Zurückziehen der Schutzhülle (14) zu greifen.

14. Verbesserte Sicherheitssubkutanspritze (117, 217) nach Anspruch 12, wobei die Verriegelungsfinger (126) gefärbt sind, um bei der Visualisierung zu helfen, dass die Schutzhülle (14) in der die Nadel abdeckenden Position arretiert ist.

15. Verbesserte Sicherheitssubkutanspritze (117, 217) nach Anspruch 12, wobei der Feststellring (22) einen Pfeil (174) auf der Oberfläche davon aufweist, um die radiale Richtung anzuzeigen, in die der Feststellring (22) zu drehen ist, um die Schutzhülle (14) permanent in der die Nadel abdeckenden Position zu arretierten.

## Revendications

1. Ressort hélicoïdal de compression/détente comprenant un ressort hélicoïdal non uniforme (117, 217) présentant plusieurs pires à 360°, chaque spire étant espacée uniformément des spires voisines, chaque spire à 360° comprenant une alternance de premières parties angulaires (140) et de seconde parties angulaires (142), les premières parties angulaires (140) définissant un premier angle par rapport à un plan perpendiculaire à un axe (20) qui traverse le centre du ressort hélicoïdal (117, 217), et les secondes parties angulaires (142) définissant un second angle par rapport au plan perpendiculaire à l'axe (20), le premier angle et le second angle étant différents.

2. Ressort hélicoïdal de compression/détente (117, 217) de la revendication 1, étant précisé que les secondes parties angulaires (142) sont des parties plates (144, 146), ces parties plates étant globalement parallèles au plan perpendiculaire à l'axe.

3. Ressort hélicoïdal de compression/détente (117, 217) de la revendication 2, étant précisé que chaque spire à 360° du ressort hélicoïdal a des première et seconde parties angulaires (140, 142) qui alternent avec des première et seconde parties plates (144, 146).

4. Ressort hélicoïdal de compression/détente (117, 217) de la revendication 3, étant précisé que le ressort hélicoïdal comprend cinq à six spires à 360°.

5. Ressort hélicoïdal de compression/détente (117, 217) de la revendication 4, étant précisé que le ressort hélicoïdal, quand il est complètement comprimé, a une longueur d'environ 2,1 cm, et a une longueur détendue d'environ 6,6 cm.

6. Ressort hélicoïdal de compression/détente (117, 217) de la revendication 4, étant précisé que le ressort, quand il est complètement comprimé, a une force de détente d'environ 5,8 N (1,3 livre).

7. Ressort hélicoïdal de compression/détente (117, 217) de la revendication 4, étant précisé que le ressort complètement comprimé, quand il est relâché, se détend pour atteindre plus de 3 fois sa longueur comprimée.

8. Seringue hypodermique de sécurité améliorée (10) comprenant :
a) un corps de seringue tubulaire creux (18),
b) une aiguille creuse (19) qui communique avec la cavité du corps de seringue (10),
c) une gaine d'aiguille tubulaire alternative (14) disposée sur l'extérieur du corps de seringue (18),
d) un ressort (117, 217) qui s'accouple au corps de seringue (10) et à la gaine (14) et qui est apte à se détendre pour déplacer la gaine (14) afin de couvrir la position d'aiguille (19), et
e) un mécanisme d'enclenchement (15) qui s'accouple au corps de seringue (10) et à la gaine (14) pour enclencher ladite gaine (14) dans une position de recouvrement d'aiguille après que le contenu a été refoulé du corps de seringue (10),
étant précisé que l'amélioration comprend un ressort hélicoïdal non uniforme (117, 217) selon l'une quelconque des revendications 2 à 7.

9. Seringue de sécurité améliorée (10) de la revendication 8, étant précisé que le ressort (117, 217) et la gaine (14) comprennent un élément d'une seule pièce.

10. Seringue de sécurité améliorée (10) de la revendication 9, étant précisé que le ressort (117, 217) et la gaine (14) sont des éléments séparés qui sont reliés pour former un élément d'une seule pièce.

11. Seringue hypodermique de sécurité améliorée (16) de la revendication 8, étant précisé que le mécanisme d'enclenchement (15) comprend une bague de verrouillage rotative (22) avec un prolongement interne (152) conçu pour s'accoupler à une rainure (37, 137, 152) prévue sur la base de la gaine (14), de manière à immobiliser des saillies d'enclenchement (126), solidaires de la gaine (14), dans une rainure (37, 137, 152) prévue sur le corps de seringue (18) près d'une extrémité formant raccord (27) de l'aiguille creuse (19).

12. Seringue hypodermique de sécurité améliorée (117, 217) de la revendication 8, étant précisé que le mécanisme d'enclenchement (15) contient une bague de verrouillage (22) qui est reliée, en les entourant, à la gaine et à la seringue, la bague de verrouillage ayant une nervure longitudinale (170) sur sa surface intérieure, ladite nervure (170) interagissant avec une rainure de passage (152) et un canal de verrouillage (152) prévus sur la base de la gaine (14) de telle sorte que la bague de verrouillage (22) est positionnée au-dessus des saillies d'enclenchement (126) solidaires de la surface de gaine (19) pour maintenir lesdites saillies d'enclenchement (126) dans une rainure (152) dans la surface de seringue à l'arrière du raccord d'aiguille (27) afin d'empêcher un mouvement de la gaine (24) une fois qu'elle est verrouillée dans la position de recouvrement d'aiguille.

13. Seringue hypodermique de sécurité améliorée (117, 217) de la revendication 12, étant précisé que la bague de verrouillage (22) comprend un bord saillant radialement sur sa surface extérieure avant, pour saisir la bague de verrouillage (22) en vue de la rétraction de la gaine (14).

14. Seringue hypodermique de sécurité améliorée (117, 217) de la revendication 12, étant précisé que les saillies d'enclenchement (126) sont de couleur pour aider à indiquer visuellement que la gaine (14) est verrouillée en position de recouvrement d'aiguille.

15. Seringue hypodermique de sécurité améliorée (117, 217) de la revendication 12, étant précisé que la bague de verrouillage (22) comprend une flèche (174), sur sa surface, pour indiquer le sens radial pour tourner la bague de verrouillage (22) afin de verrouiller de manière permanente la gaine (14) dans sa position de recouvrement d'aiguille.
